# EUROPEAN PATENT SPECIFICATION

(11) **EP 1 600 104 B1**
(45) Date of publication and mention of the grant of the patent: **20.04.2011**
(21) Application number: 05107977.0
(22) Date of filing: 10.10.2003
(51) Int. Cl.: A61B 5/053, A61B 5/00, A61B 5/103

(54) **Determination of biological conditions using impedance measurements**
Bestimmung von biologischen Konditionen mittels Impedanzmessungen
Détermination de propriétés biologiques par mesure de l'impédance

(30) Priority: 11.10.2002 US 417561 P
(43) Date of publication of application: 30.11.2005
(62) Divisional of application: 03023156.7
(73) Proprietor: SciBase AB, 111 40 Stockholm (SE)
(72) Inventor: Ollmar, Stig, 141 41 Huddinge (SE); Åberg, Peter, 531 36 Lidköping (SE); Nicander, Ingrid, 11633 Stockholm (SE)
(74) Representative: Presland, Torbjörn

(56) References cited:
- WO-A-01/52731
- WO-A-92/06634
- EMTESTAM ET AL: "Electrical Impedance of Nodular Basal Cell Carcinoma: a pilot study" DERMATOLOGY, vol. 197, 1998, pages 313-316, XP008026174
- GRISS P ET AL: "MICROMACHINED ELECTRODES FOR BIOPOTENTIAL MEASUREMENTS" JOURNAL OF MICROELECTROMECHANICAL SYSTEMS, IEEE INC. NEW YORK, US, vol. 10, no. 1, March 2001 (2001-03), pages 10-16, XP001123702 ISSN: 1057-7157
- OLLMAR S ET AL: "ELECTRICAL IMPEDANCE FOR ESTIMATION OF IRRITATION IN ORAL MUCOSA AND SKIN" MEDICAL PROGRESS THROUGH TECHNOLOGY, SPRINGER VERLAG. BERLIN, DE, vol. 21, no. 1, 1 February 1995 (1995-02-01), pages 29-37, XP000502787 ISSN: 0047-6552

## Description

### FIELD OF THE INVENTION

The present invention generally relates to the field of diagnosis of biological conditions, and in particular to a medical device and a method for non-invasively diagnosing a diseased condition of the skin of a subject, particularly the presence of skin cancer, e.g. basal cell carcinoma or malignant melanoma, a squamous cell carcinoma or precursors thereof, using skin impedance measurements.

### BACKGROUND OF THE INVENTION

Basal cell carcinoma (BCC) is the most common skin cancer. Its incidence is increasing in many countries throughout the world, for example, in Sweden. Long-term immunosuppression, e.g. after allogeneic organ transplantation, increases the risk of developing BCC and other skin tumours. This is also the case following exposure to ultraviolet light or ionizing radiation. There seems to be no apparent genetic connection and in many patients no other predisposing factors are found. However, clinical diagnosis of skin tumours can prove difficult even for experienced dermatologists, especially in the case of pigmented lesions. In the clinic there is a need for a diagnostic aid besides the established naked eye in combination with skin biopsies for histological examination.

Non-invasive methods of making biological determinations, such as clinical diagnosis of skin tumours, are generally desirable over invasive techniques that involve the taking of samples. Non-invasive techniques can be more convenient, e.g. less painful, involve less risk of infection etc. Accordingly, a number of non-invasive techniques for making biological determinations have been proposed:

| Application No. | Publication No | Publication date | Name |
|---|---|---|---|
| | US 5,036,861 | August 6, 1991 | Sembrowich *et al.* |
| | US 5,115,133 | May 19,1992 | Knudson |
| | US 5,146,091 | September 8, 1992 | Knudson |
| | US 5,197,951 | January 19, 1993 | Knudson. |
| | US 5,222,496 | June 29, 1993 | Clarke *et al*. |
| PCT/US94/08816 | WO95/04496 | February 16, 1995 | Solid State Farms, inc. |
| | US 5,433,197 | July 18, 1995 | Stark |
| PCT/US97/13267 | WO 98/04190 | February 5, 1998 | Dermal Therapy (Barbados) Inc. |
| PCT/US98/02037 | WO 99/39627 | August 12, 1999 | Dermal Therapy (Barbados) Inc. |
| PCT/IB00/01464 | WO 01/26338 | October 13, 2000 | Süsstrunk, *et al.* |

However, all of the above-mentioned documents disclose techniques for evaluating blood glucose levels and are consequently not suitable for diagnosing a diseased condition of the skin of a subject, particularly the presence of skin cancer, e.g. basal cell carcinoma.

Electrical impedance has been found to be a very sensitive indicator of minute changes in organic and biological material and especially tissues such as mucous membranes, skin and integuments of organs and, hence, provides an effective tool for non-invasive measurements of variations in structural properties of the tissue. In PCT/SE91/00703 a device for non-invasive measurement of electrical impedance of organic and biological material is disclosed. A probe comprising a number of electrodes arranged in a concentric ring system. The electrodes are driven from a control unit in such a way that the electric current path defining the actual tissue under test is dependent upon a control signal, is pressed towards the surface of the body part under test. By varying the control signal it is possible to select the region under test. Two rings supply voltage and the relation between the two will generate a virtual projection point located between the two rings. By adjusting the voltage relation between the two injection points the virtual injection point can be moved back and forth and hence the depth penetration in the tissue is selectable. However, human skin is a complex, heterogeneous, and anisotropic multilayer structure with electronically non-linear properties. The most non-linear properties are located to the Stratum Corneum (the outermost layer of epidermis). Therefore, there is information in all depth measurement that cannot be calculated by interpolation or extrapolation from two depths although the different depths are highly correlated. However, the device disclosed in PCT/SE91/00703 is impaired with a severe drawback in that it cannot deliver reliable results regarding tissue variation in skin layers beneath stratum corneum where, for example, skin cancer and allergic reactions manifest. This is due to the fact that non-invasive electrical impedance spectra of skin are dominated by the dielectric properties of the Stratum Corneum, especially at low frequencies. The stratum corneum has properties (a large and broad so called alpha dispersion) that may lead to that responses from underlying viable skin layers is confounded with responses from stratum corneum, thus diluting the clinically relevant information from the viable skin. This makes it difficult and unpredictable to assess electrical impedance phenomena that manifest below the stratum corneum using the probe disclosed in PCT/SE91/00703.

Accordingly, at the present, a reliable non-invasive method for diagnosing a diseased condition of the skin of a subject, particularly the presence of skin cancer, e.g. basal cell carcinoma or malignant melanoma, a squamous cell carcinoma or precursors thereof is lacking.

### BRIEF DESCRIPTION OF THE INVENTION

Thus, an object of the present invention is to provide an apparatus and a method for diagnosing a diseased condition of the skin of a subject, particularly the presence of skin cancer, e.g. basal cell carcinoma or malignant melanoma, a squamous cell carcinoma or precursors thereof in an accurate and reliable way.

This and other objects are achieved according to the present invention by providing a medical apparatus and a method having the features defined in the independent claims. Preferable embodiments of the invention are characterised by the dependent claims.

According to an aspect of the present invention, there is provided an apparatus for the diagnosing a diseased condition of the skin of a subject, comprising an electrically conducting probe including a plurality of electrodes, each electrode comprising at least one spike, which spikes are laterally spaced apart from each other and having a length being sufficient to penetrate the stratum corneum, wherein the probe is adapted to be placed against a skin surface of the subject such that the spikes penetrate the stratum corneum. The apparatus is adapted to pass an electrical current through the electrodes to obtain values of skin impedance and to use reference data to determine whether the obtained impedance values indicate the diseased condition.

According to a second aspect of the present invention, there is provided a method for diagnosing a diseased condition of the skin of a subject, comprising the steps of:
placing an electrically conducting probe against a skin surface of the subject, the probe including a plurality of electrodes, each electrode comprising at least one spike, which spikes are laterally spaced apart from each other and having a length being sufficient to penetrate the stratum corneum, passing an electrical current through the electrodes to obtain a value of skin impedance; and using reference data to determine whether the obtained impedance values indicate the diseased condition.

Thus, the invention is based on the insight that the non-invasive electrical impedance spectra of skin are dominated by the dielectric properties of the Stratum Corneum, especially at low frequencies. The stratum corneum has properties (a large and broad so called alpha dispersion) that may lead to that responses from underlying viable skin layers is confounded with responses from stratum corneum, thus diluting the clinically relevant information from the viable skin.

This invention provides several advantages in comparison to the known technique disclosed in, for example, PCT/SE91/00703. As an example, electrical impedance phenomena that manifest below the stratum corneum can be assessed in a reliable manner using the present invention. Thereby, by using the present invention, a diseased condition of the skin of a subject, particularly the presence of skin cancer, e.g. basal cell carcinoma or malignant melanoma, a squamous cell carcinoma or precursors thereof can be diagnosed in an accurate and reliable way.

The features that characterize the invention, both as to organization and to method of operation, together with further objects and advantages thereof, will be better understood from the following description used in conjunction with the accompanying drawings. It is to be expressly understood that the drawings is for the purpose of illustration and description and is not intended as a definition of the limits of the invention. These and other objects attained, and advantages offered, by the present invention will become more fully apparent as the description that now follows is read in conjunction with the accompanying drawings.

### BRIEF DESCRIPTION OF THE DRAWINGS

In the following detailed description, reference will be made to the accompanying drawings, of which:
Fig. 1(a) shows an embodiment of an electrode furnished with spikes according to the present invention;
Fig. 1(b) shows details of the spiked array given as electron micrograph;
Fig. 2 shows representative Bode plots of impedance (left hand axis; KOhm) and phase angle (right hand axis; degrees) as a function of frequency (kHz), obtained at five depth settings using a spiked electrode. In Fig. 2(a), the results were obtained for a normal skin site of a subject. In
Fig. 2(b), the results were obtained from the same subject but as a basal cell carcinoma near the normal site of Fig. 2(a). In Fig. 2(c), the results were obtained from a normal skin site of another subject. In Fig. 2(d), the results were obtained from this other subject but a malignant melanoma located near the normal site of Fig. 2(c). Each ensemble of curves represents five measured depths.

### DETAILED DESCRIPTION OF THE INVENTION

The spikes are laterally spaced apart from each other and having a length being sufficient to penetrate the stratum corneum. The probe is adapted to be placed against a skin surface of the subject such that the spikes penetrate the stratum corneum. Preferably, the skin surface sites are soaked prior to each impedance measurement using, for example, a 0.9 % saline solution. The sites can be soaked, for example, for 60 seconds prior to the measurement.

The apparatus according to the present invention is adapted to pass an electrical current through the electrodes to obtain a value of skin impedance and to use reference data to determine whether the obtained impedance value indicates the diseased condition. The electrical current has a frequency between 1 KHz and 1 MHz. Preferably, each spike has a length being sufficient to penetrate below the skin surface to the Stratum Germinativum or through the Stratum Corneum, i.e. through the Stratum Corneum into the living Epidermis. Preferably, each spike has a length between about 10 µm and about 250 µm and an outer diameter between about 20 µm and about 50 µm.

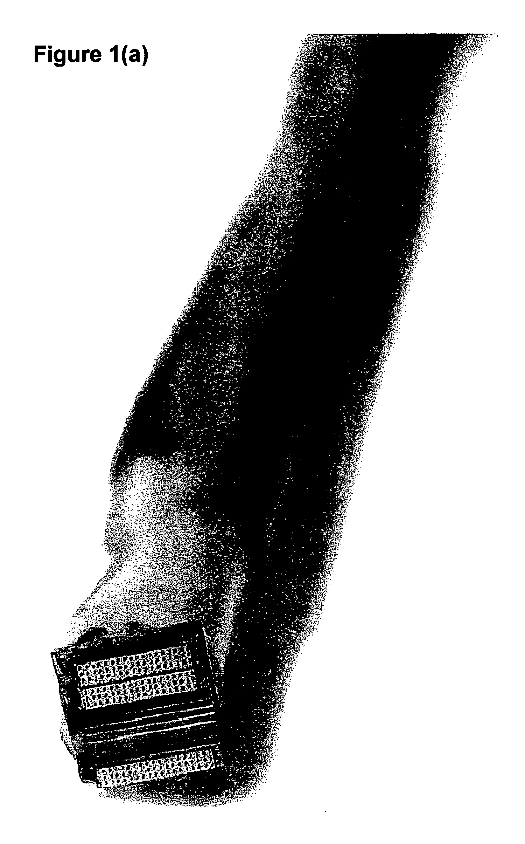

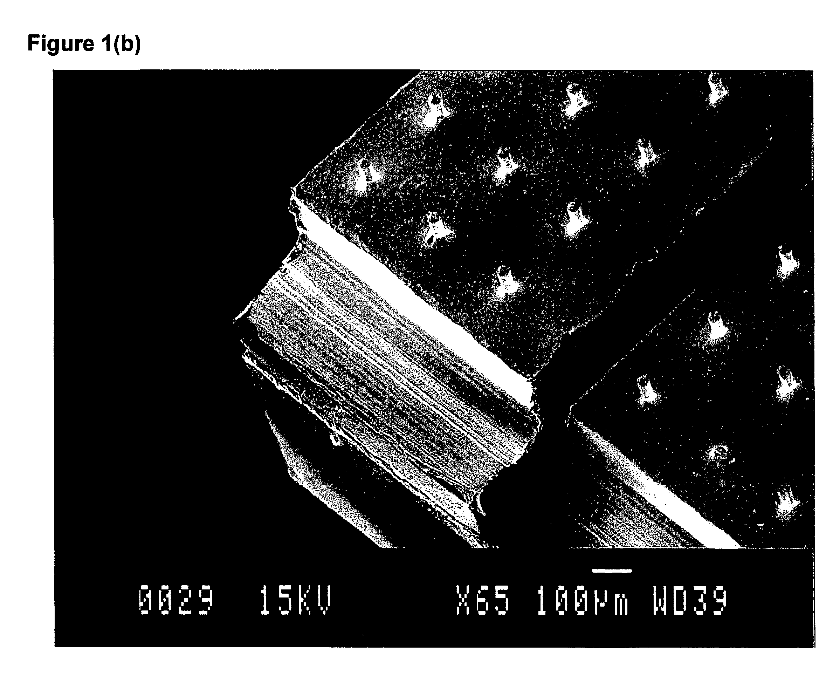

An apparatus for use according to the present invention can generally be regarded as a combination of the device described in international patent application No. PCT/SE91/00703, published under WO 92/06634 on April 30, 1992 and the "spiked" electrode described in international patent application No. PCT/ IB01/00059, published under WO 01/52731 on July 26, 2001 or in an article entitled "Micromachined electrodes for biopotential measurements" published in the Journal of Microelectromechanical Systems 10(1), pp 10-16, on March 2001 by Griss et al. The electrode used in the tests described below, however, is a variation of the described by Griss et al., and is shown in Figs. 1(a) and 1(b). The probe includes a number of electrodes, at least three according to PCT/SE91/00703 and in the present invention each electrode is provided with at least one spike, thereby forming a spiked surface, which permits measurements to be made at a variety of skin depths. The probe is illustrated in Fig. 1 (b), the probe being viewed looking down onto its spikes (a bottom plan view). The probe includes three rectangular areas or bars, each bar containing an array of 35 (7 x 5) spikes. Each bar is 1 mm wide and 5 mm long. The distance between the closest bars is 0.2 mm, and the distance between the second and the third bars is 1.8 mm. The active part of the probe is thus about 5 x 5 mm. Each spike has a length of approximately 150 micrometer, as measured from its base, and a thickness of approximately 25 micrometer. The spikes are sharpened cylinders, i.e. are needle-like, and spaced approximately 200 micrometers from each other, center to center. The spikes are preferably made of silicon and covered with gold having a thickness of approximately 2 micrometer. Any material comprising a conductive surface with similar dimensions would work, but should be selected to be bio-compatible.

The apparatus without the spiked probe is known as the SciBase II depth selective spectrometer, may be obtained from SciBase AB of Stockholm, Sweden. The pin assignment for the probe connected is as follows:
1. <START> button.
2. sense (first electrode illustrated in Fig. 1(b); use coaxial (conventional probe) screen 3).
3. gnd (for sense).
4. near exciter (second (middle) electrode illustrated in Fig. 1(b); use coaxial (conventional probe) screen 5).
5. gnd (for near injection).
6. gnd.
7. far exciter (third (right-most) electrode illustrated in Fig. 1(b); use coaxial (conventional probe) screen 8).
8. gnd (for far injection).
9. chassis.
10. reserved.
11. reserved.
12. gnd.
13. gnd.
14. charger.

Preferably, the impedance was measured using the SciBase II depth selective spectrometer at 31 logarithimically distributed frequencies from 1 kHz to 1 MHz at five depth settings, as described in PCT/SE/00703.

The probe shown in Fig. 1(b) is an embodiment of the present invention and hereinafter other embodiments will be discussed.

Each spike may be at least 20, or at least 30, or at least 40, or at least 50, or at least 60, or at least 70, or at least 80, or at least 90 µm in length.

According to an alternative embodiment, each spike has a thickness of about 30 micrometer.

Additionally, each spike may be up to 250, or up to 240, or up to 230, or up to 220, or up to 210, or up to 200, or up to 190, or up to 180, or up to 170, or up to 160, or up to 150, or up to 140, or up to 130, or up to 120, or up to 110, or up to 100 µm in length.

According to the invention, the probe comprises three electrodes, wherein the spikes of a first electrode and a second electrode being laterally spaced apart a first distance from each other and the spikes of the first and a third electrode being laterally spaced apart a second distance from each other. The first distance and the second distance are different from each other. The first distance is between about 0.1 mm and about 40 mm and the second distance is between about 1 mm and about 50 mm. The apparatus is adapted to separately pass an electrical current between the first and the second electrode and the first and the third electrode to obtain first and second values of skin impedance. Specifically, the first distance is between about 0.1 mm and about 40 mm, or between about 0.1 mm and about 30 mm, or between about 0.1 mm and about 25 mm, or between about 0.1 mm and about 20 mm, or between about 0.1 mm and about 15 mm, or between about 0.2 mm and about 10 mm, or between about 0.2 mm and about 5 mm, or between about 0.2 mm and about 3 mm, or between about 0.2 mm and about 2 mm, or between about 0.2 mm and about 1.5 mm, or between about 0.2 mm and about 1 mm, or between about 0.2 mm and about 0.5 mm. In addition, the second distance is between about 1 mm and about 50 mm, or between about 1 mm and about 40 mm, or between about 1 mm and about 30 mm, or between about 1 mm and about 15 mm, or between about 1 mm and about 10 mm, or between about 1 mm and about 9 mm, or between about 1 mm and about 8 mm, or between about 1 mm and about 7 mm, or between about 2 mm and about 8 mm, or between about 3 mm and about 7 mm, or between about 4 mm and about 7 mm, or between about 4 mm and about 6 mm, or about 5 mm.

Furthermore, each electrode may comprise at least two spikes, or at least three spikes, or at least four spikes, or at least five spikes, or at least six spikes, or at least seven spikes, or at least eight spikes, or at least nine spikes, or at least ten spikes, or at least twelve spikes, or at least fifteen spikes, or at least eighteen spikes, or at least twenty spikes, or at least twenty-five spikes, or at least thirty spikes, or at least thirty-five spikes, or at least fifty spikes. As mentioned above, the probe illustrated in Fig. 1(b) includes three rectangular areas or bars, each bar containing an array of 35 (7 x 5) spikes. However, as the man skilled within the art realizes, other configurations may be used, for example, bars containing an array of 57 (19 x 3) spikes.

### Cancer diagnosis

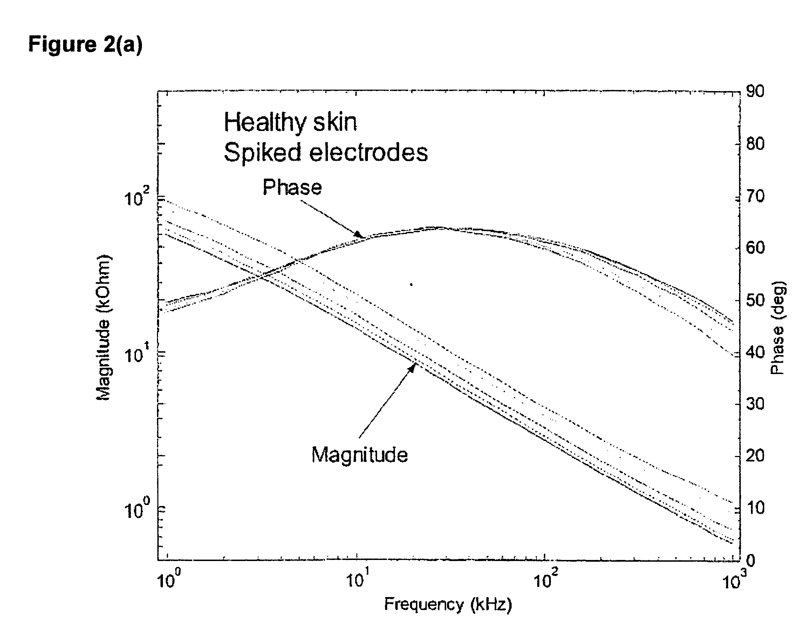

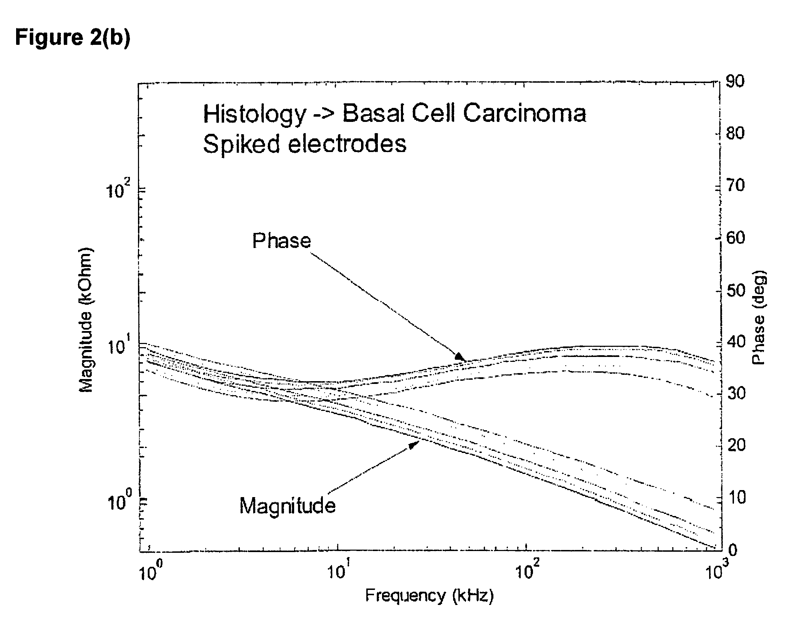

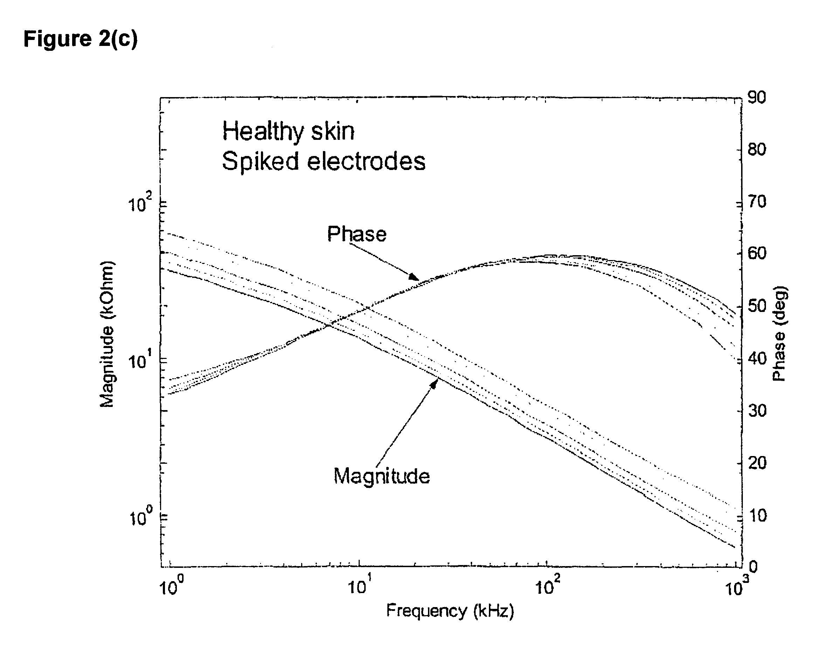

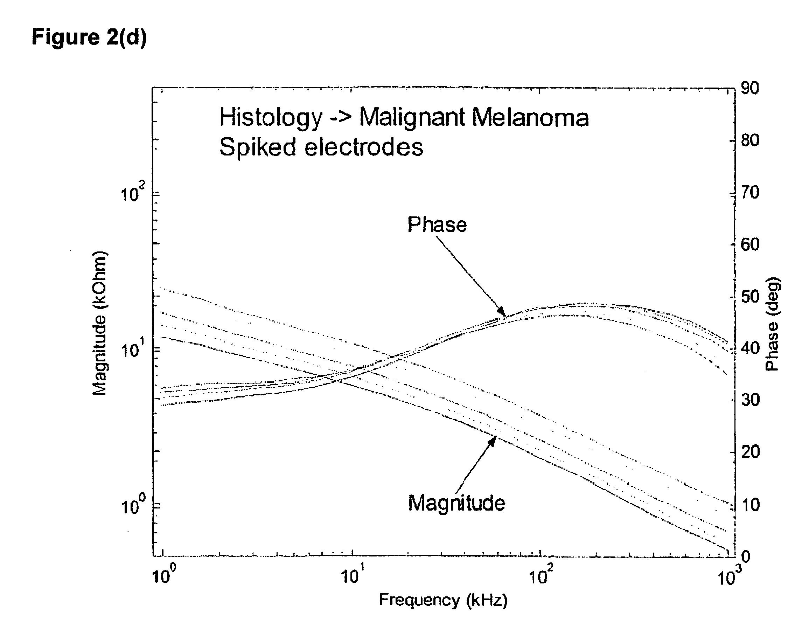

Impedance measurements were taken from subject suffering from basal cell carcinoma or malignant melanoma: at a first site of normal (unaffected skin); and at a second site of diseased skin. Results obtained are shown in Fig. 2. A further description of the approach, in which measurements were obtained using a conventional probe, is given in Emtestam I, Nicander I, Stenström M, Ollmar S. "Electrical impedance of nodular basal cell carcinoma: a pilot study", Dermatology 1998; 197: 313-316, and Kapoor S. "Bioelectric impedance techniques for clinical detection of skin cancer using simple electrical impedance indices", Skin Res Technol 2003; 9: 257-261, and Beetner DG, Kapoor S, Manjunath S, Zhou X, Stoecker WV "Differentation among basal cell carcinoma, benign lesions, and normal skin using electric impedance", IEEE Trans Biomed Eng 2003; 50: 1020-1025.

It is desirable to detect and remove skin cancers as early as possible. As such, precursors of skin cancer, such as, for example, actinic keratose (a precursor of squamous cell carcinoma) and dysplastic nevi (a precursor of malignant melanoma), as well as other lesions that may be mixed up with various cancers unless surgery and histological evaluation of the catch is made, can be detected using impedance measurements of the present invention in the manner described herein.

Although exemplary embodiments of the present invention has been shown and described, it will be apparent to those having ordinary skill in the art that a number of changes, modifications, or alterations to the inventions as described herein may be made. Thus, it is to be understood that the above description of the invention and the accompanying drawings is to be regarded as a non-limiting example thereof and that the scope of protection is defined by the appended patent claims.

## Claims

1. An apparatus for the diagnosing of a diseased condition of the skin of a subject, comprising:
an electrically conducting probe including plurality of electrodes, each electrode comprising at least one spike, which spikes are laterally spaced apart from each other and having a length being sufficient to penetrate the stratum corneum, and wherein
said probe is adapted to be placed against a skin surface of the subject such that said spikes penetrate the stratum corneum, said apparatus being adapted to pass an electrical current through the electrodes to obtain values of skin impedance, wherein said electrical current has a frequency between 1 kHz and 1 MHz, and to use reference data to determine whether the obtained impedance values indicate the diseased condition.

2. The apparatus according to claim 1, wherein the diseased condition is cancer, preferably skin cancer.

3. The apparatus according to claim 2, wherein skin cancer is, a malignant melanoma, or precursors of such lesion.

4. The apparatus according to any one of preceding claims, wherein the apparatus is adapted to pass the electrical current at a plurality of logarithmically distributed frequencies within the range from 1 kHz to 1 MHz.

5. The apparatus according to any one of preceding claims, wherein each of said spikes has a length being sufficient to penetrate below the skin surface to the Stratum Germinativum or through the Stratum Corneum into the living Epidermis but not into the Dermis.

6. The apparatus according to any one of preceding claims, wherein said apparatus is adapted to use both non-invasive surface electrodes (conventional probes) in conjunction with said spiked electrodes to obtain more aspects of skin properties in order to improve power of discrimination.

7. The apparatus according to any one of preceding claims, wherein each spike has a length of at least about 10 µm.

8. The apparatus according to any one of preceding claims, wherein said probe comprises three said spike furnished electrodes, the spikes of a first electrode and a second electrode being laterally spaced apart a first distance from each other and the spikes of the first and a third electrode being laterally spaced apart a second distance from each other, said apparatus being adapted to separately pass an electrical current between the first and the second electrode and the first and the third electrode to obtain first and second values of skin impedance or a mixture of values of skin impedance between values obtained using the first and second electrode and values obtained using the first and third electrode.

9. The apparatus according to claim 8, wherein said first distance and said second distance are different from each other.

10. The apparatus according to claim 8 or 9, wherein said first distance is between about 0.1 mm and about 40 mm.

11. The apparatus according to claim 8, 9 or 10, wherein said second distance is between about 1 mm and 50 mm.

12. The apparatus according to any one of preceding claims, wherein each of said spikes has a length up to about 250 µm.

13. The apparatus according to any one of preceding claims, wherein an outer diameter of each of said spikes is between about 20 µm and about 50 µm

14. A method for diagnosing of a diseased condition of the skin of a subject, comprising the steps of:
placing an electrically conducting probe against a skin surface of the subject, said probe including plurality of electrodes, each electrode comprising at least one spike, which spikes are laterally spaced apart from each other and having a length being sufficient to penetrate the stratum corneum,
passing an electrical current having a frequency between 1 kHz and 1 MHz through the electrodes to obtain values of skin impedance, and
using, by means of an apparatus, reference data to determine whether the obtained impedance values indicate the diseased condition.

15. The method according to claim 14, wherein the diseased condition is cancer, preferably skin cancer.

16. The method according to claim 15, wherein skin cancer is a malignant melanoma or precursors of such lesion.

17. The method according to any one of preceding claims 14-16, wherein each of said spikes has a length being sufficient to penetrate below the skin surface to the Stratum Germinativum or through the Stratum Corneum into the living Epidermis but not into the Dermis.

18. The method according to any one of preceding claims 14-17, wherein non-invasive surface electrodes (conventional probes) are used in conjunction with said spiked electrodes to obtain more aspects of skin properties in order to improve power of discrimination.

19. The method according to any one of preceding claims 14-18, wherein each spike has a length of at least about 10 µm.

20. The method according to any one of preceding claims 14-19, wherein said probe comprises three said spike furnished electrodes, the spikes of a first electrode and a second electrode being laterally spaced apart a first distance from each other and the spikes of the first and a third electrode being laterally spaced apart a second distance from each other, wherein the step of passing an electrical current comprises the step of separately passing an electrical current between the first and the second electrode and the first and the third electrode to obtain first and second values of skin impedance or a mixture of values of skin impedance between values obtained using the first and second electrode and values obtained using the first and third electrode.

21. The method according to claim 20, wherein said first distance and said second distance are different from each other.

22. The method according to claim 20 or 21, wherein said first distance is between about 0.1 mm and about 40 mm.

23. The method according to claim 22, wherein said second distance is between about 1 mm and 50 mm.

24. The method according to any one of preceding claims 14-23, wherein each of said spikes has a length up to about 250 µm.

25. The method according to any one of preceding claims 14-24, wherein an outer diameter of each of said spikes is between about 20 µm and about 50 µm.

26. The method according to any one of preceding claims 14-25, wherein said passing comprises passing said electrical current through the electrodes at a plurality of logarithmically distributed frequencies within a range from 1 kHz to 1 MHz.

## Patentansprüche

1. Vorrichtung zum Diagnostizieren eines Erkrankungszustandes der Haut eines Subjekts, wobei die Vorrichtung Folgendes umfasst:
eine elektrisch leitende Sonde, die mehrere Elektroden einschließt, wobei jede Elektrode wenigstens einen Dorn umfasst, wobei diese Dorne mit seitlichem Abstand entfernt voneinander angeordnet sind und eine Länge haben, die ausreichend ist, um das Stratum corneum zu durchdringen, und wobei
die Sonde dafür eingerichtet ist, an einer Hautoberfläche des Subjekts angeordnet zu werden derart, dass die Dorne das Stratum corneum durchdringen, wobei die Vorrichtung dafür eingerichtet ist, einen elektrischen Strom durch die Elektroden hindurchzuleiten, um Werte der Hautimpedanz zu erlangen, wobei der elektrische Strom eine Frequenz zwischen 1 kHz und 1 MHz hat, und Referenzdaten zu verwenden, um festzustellen, ob die erlangten Impedanzwerte auf den Erkrankungszustand hinweisen.

2. Vorrichtung nach Anspruch 1, wobei der Erkrankungszustand Krebs, vorzugsweise Hautkrebs, ist.

3. Vorrichtung nach Anspruch 2, wobei der Hautkrebs ein malignes Melanom oder Vorläufer einer solchen Läsion ist.

4. Vorrichtung nach einem der vorhergehenden Ansprüche, wobei die Vorrichtung dafür eingerichtet ist, den elektrischen Strom mit mehreren logarithmisch verteilten Frequenzen innerhalb des Bereichs von 1 kHz bis 1 MHz hindurchzuleiten.

5. Vorrichtung nach einem der vorhergehenden Ansprüche, wobei jeder der Dorne eine Länge hat, die ausreichend ist, um unter die Hautoberfläche bis zum Stratum germinativum oder durch das Stratum corneum in die lebende Epidermis, aber nicht in die Dermis, einzudringen.

6. Vorrichtung nach einem der vorhergehenden Ansprüche, wobei die Vorrichtung dafür eingerichtet ist, sowohl nicht invasive Oberflächenelektroden (herkömmliche Sonden) in Verbindung mit den mit Dornen versehenen Elektroden zu verwenden, um mehr Aspekte von Hauteigenschaften zu erlangen, um das Unterscheidungsvermögen zu verbessern.

7. Vorrichtung nach einem der vorhergehenden Ansprüche, wobei jeder Dorn eine Länge von wenigstens etwa 10 µm hat.

8. Vorrichtung nach einem der vorhergehenden Ansprüche, wobei die Sonde drei mit Dornen ausgestattete Elektroden umfasst, wobei die Dorne der ersten Elektrode und einer zweiten Elektrode mit einem ersten seitlichen Abstand entfernt voneinander angeordnet sind und die Dorne der ersten Elektrode und einer dritten Elektrode mit einem zweiten seitlichen Abstand entfernt voneinander angeordnet sind, wobei die Vorrichtung dafür eingerichtet ist, gesondert einen elektrischen Strom zwischen der ersten und der zweiten Elektrode und der ersten und der dritten Elektrode hindurchzuleiten, um erste und zweite Werte der Hautimpedanz oder eine Mischung von Werten der Hautimpedanz zwischen unter Verwendung der ersten und der zweiten Elektrode erlangten Werten und unter Verwendung der ersten und der dritten Elektrode erlangten Werten zu erlangen.

9. Vorrichtung nach Anspruch 8, wobei sich der erste Abstand und der zweite Abstand voneinander unterscheiden.

10. Vorrichtung nach Anspruch 8 oder 9, wobei der erste Abstand zwischen etwa 0,1 mm und etwa 40 mm beträgt.

11. Vorrichtung nach Anspruch 8, 9 oder 10, wobei der zweite Abstand zwischen etwa 1 mm und 50 mm beträgt.

12. Vorrichtung nach einem der vorhergehenden Ansprüche, wobei jeder der Dorne eine Länge von bis zu etwa 250 µm hat.

13. Vorrichtung nach einem der vorhergehenden Ansprüche, wobei ein Außendurchmesser jedes der Dorne zwischen etwa 20 µm und etwa 50 µm beträgt.

14. Verfahren zum Diagnostizieren eines Erkrankungszustandes der Haut eines Subjekts, wobei das Verfahren die folgenden Schritte umfasst:
das Anordnen einer elektrisch leitenden Sonde an einer Hautoberfläche des Subjekts, wobei die Sonde mehrere Elektroden umfasst, wobei jede Elektrode wenigstens einen Dorn umfasst, wobei diese Dorne mit seitlichem Abstand entfernt voneinander angeordnet sind und eine Länge haben, die ausreichend ist, um das Stratum corneum zu durchdringen,
das Hindurchleiten eines elektrischen Stroms, der eine Frequenz zwischen 1 kHz und 1 MHz hat, durch die Elektroden, um Werte der Hautimpedanz zu erlangen, und
das Verwenden von Referenzdaten, mit Hilfe einer Vorrichtung, um festzustellen, ob die erlangten Impedanzwerte auf den Erkrankungszustand hinweisen.

15. Verfahren nach Anspruch 14, wobei der Erkrankungszustand Krebs, vorzugsweise Hautkrebs, ist.

16. Verfahren nach Anspruch 15, wobei der Hautkrebs ein malignes Melanom oder Vorläufer einer solchen Läsion ist.

17. Verfahren nach einem der vorhergehenden Ansprüche 14 bis 16, wobei jeder der Dorne eine Länge hat, die ausreichend ist, um unter die Hautoberfläche bis zum Stratum germinativum oder durch das Stratum corneum in die lebende Epidermis, aber nicht in die Dermis, einzudringen.

18. Verfahren nach einem der vorhergehenden Ansprüche 14 bis 17, wobei nicht invasive Oberflächenelektroden (herkömmliche Sonden) in Verbindung mit den mit Dornen versehenen Elektroden verwendet werden, um mehr Aspekte von Hauteigenschaften zu erlangen, um das Unterscheidungsvermögen zu verbessern.

19. Verfahren nach einem der vorhergehenden Ansprüche 14 bis 18, wobei jeder Dorn eine Länge von wenigstens etwa 10 µm hat.

20. Verfahren nach einem der vorhergehenden Ansprüche 14 bis 19, wobei die Sonde drei mit Dornen ausgestattete Elektroden umfasst, wobei die Dorne der ersten Elektrode und einer zweiten Elektrode mit einem ersten seitlichen Abstand entfernt voneinander angeordnet sind und die Dorne der ersten Elektrode und einer dritten Elektrode mit einem zweiten seitlichen Abstand entfernt voneinander angeordnet sind, wobei der Schritt des Hindurchleitens eines elektrischen Stroms den Schritt des gesonderten Hindurchleitens eines elektrischen Stroms zwischen der ersten und der zweiten Elektrode und der ersten und der dritten Elektrode umfasst, um erste und zweite Werte der Hautimpedanz oder eine Mischung von Werten der Hautimpedanz zwischen unter Verwendung der ersten und der zweiten Elektrode erlangten Werten und unter Verwendung der ersten und der dritten Elektrode erlangten Werten zu erlangen.

21. Verfahren nach Anspruch 20, wobei sich der erste Abstand und der zweite Abstand voneinander unterscheiden.

22. Verfahren nach Anspruch 20 oder 21, wobei der erste Abstand zwischen etwa 0,1 mm und etwa 40 mm beträgt.

23. Verfahren nach Anspruch 22, wobei der zweite Abstand zwischen etwa 1 mm und 50 mm beträgt.

24. Verfahren nach einem der vorhergehenden Ansprüche 14 bis 23, wobei jeder der Dorne eine Länge von bis zu etwa 250 µm hat.

25. Verfahren nach einem der vorhergehenden Ansprüche 14 bis 24, wobei ein Außendurchmesser jedes der Dorne zwischen etwa 20 µm und etwa 50 µm beträgt.

26. Verfahren nach einem der vorhergehenden Ansprüche 14 bis 25, wobei das Hindurchleiten das Hindurchleiten des elektrischen Stroms mit mehreren logarithmisch verteilten Frequenzen innerhalb des Bereichs von 1 kHz bis 1 MHz umfasst.

## Revendications

1. Appareil pour le diagnostic d'un état pathologique de la peau d'un sujet, comprenant :
une sonde conductrice électrique incluant une pluralité d'électrodes, chaque électrode comprenant au moins une aiguille, lesquelles aiguilles étant latéralement espacées les unes des autres et ayant une longueur suffisante pour pénétrer dans le stratum corneum, et dans lequel
ladite sonde est apte à être placée contre une surface de peau du sujet de manière à ce que lesdites aiguilles pénètrent dans le stratum corneum, ledit appareil étant apte à faire passer un courant électrique dans les électrodes pour obtenir des valeurs d'impédance de la peau, ledit courant électrique ayant une fréquence comprise entre 1 kHz et 1 MHz, et à utiliser des données de référence pour déterminer si les valeurs d"impédance obtenues indiquent l'état pathologique.

2. Appareil selon la revendication 1, dans lequel l'état pathologique est le cancer, de préférence le cancer de la peau.

3. Appareil selon la revendication 2, dans lequel le cancer de la peau est un mélanome malin ou des précurseurs de cette lésion.

4. Appareil selon l'une quelconque des revendications précédentes, dans lequel l'appareil est apte à faire passer le courant électrique à une pluralité de fréquences réparties de manière logarithmique dans la plage de 1 kHz à 1 MHz.

5. Appareil selon l'une quelconque des revendications précédentes, dans lequel chacune desdites aiguilles a une longueur suffisante pour pénétrer sous la surface de la peau jusqu'au stratum germinativum ou dans le stratum corneum dans l'épiderme vivant mais pas dans le derme.

6. Appareil selon l'une quelconque des revendications précédentes, dans lequel ledit appareil est apte à utiliser à la fois des électrodes de surface non invasives (sondes conventionnelles) en conjonction avec lesdites électrodes à aiguilles pour obtenir plus d'aspects de propriétés de la peau afin d'améliorer la capacité de discrimination.

7. Appareil selon l'une quelconque des revendications précédentes, dans lequel chaque aiguille a une longueur d'au moins environ 10 µm.

8. Appareil selon l'une quelconque des revendications précédentes, dans lequel ladite sonde comprend trois dites électrodes équipées d'aiguilles, les aiguilles d'une première électrode et d'une deuxième électrode étant espacées latéralement d'une première distance les unes des autres et les aiguilles des première et troisième électrodes étant espacées latéralement d'une deuxième distance les unes des autres, ledit appareil étant apte à faire passer séparément un courant électrique entre les première et deuxième électrodes et les première et troisième électrodes pour obtenir des première et deuxième valeurs d'impédance de la peau ou un mélange de valeurs d'impédance de la peau entre les valeurs obtenues en utilisant les première et deuxième électrodes et les valeurs obtenues en utilisant les première et troisième électrodes.

9. Appareil selon la revendication 8 , dans lequel ladite première distance et ladite deuxième distance sont différentes l'une de l'autre.

10. Appareil selon la revendication 8 ou 9, dans lequel ladite première distance est comprise entre environ 0,1 mm et environ 40 mm.

11. Appareil selon la revendication 8, 9 ou 10, dans lequel ladite deuxième distance est comprise entre environ 1 mm et 50 mm.

12. Appareil selon l'une quelconque des revendications précédentes, dans lequel chacune desdites aiguilles a une longueur allant jusqu'à environ 250 µm.

13. Appareil selon l'une quelconque des revendications précédentes, dans lequel un diamètre extérieur de chacune desdites aiguilles est compris entre environ 20 µm et environ 50 µm.

14. Procédé de diagnostic d'un état pathologique de la peau d'un sujet, comprenant les étapes consistant à :
placer une sonde conductrice électrique contre une surface de peau du sujet, ladite sonde incluant une pluralité d'électrodes, chaque électrode comprenant au moins une aiguille, lesquelles aiguilles étant latéralement espacées les unes des autres et ayant une longueur suffisante pour pénétrer dans le stratum corneum,
faire passer un courant électrique ayant une fréquence comprise entre 1 kHz et 1 MHz dans les électrodes pour obtenir des valeurs d'impédance de la peau, et
utiliser, au moyen d'un appareil, les données de référence pour déterminer si les valeurs d"impédance obtenues indiquent l'état pathologique.

15. Procédé selon la revendication 14, dans lequel l'état pathologique est le cancer, de préférence le cancer de la peau.

16. Procédé selon la revendication 15, dans lequel le cancer de la peau est un mélanome malin ou des précurseurs de cette lésion.

17. Procédé selon l'une quelconque des revendications précédentes 14 à 16, dans lequel chacune desdites aiguilles a une longueur suffisante pour pénétrer sous la surface de la peau jusqu'au stratum germinativum ou dans le stratum corneum dans l'épiderme vivant mais pas dans le derme.

18. Procédé selon l'une quelconque des revendications précédentes 14 à 17, dans lequel on utilise des électrodes de surface non invasives (sondes conventionnelles) en conjonction avec lesdites électrodes à aiguilles pour obtenir plus d'aspects de propriétés de la peau afin d'améliorer la capacité de discrimination.

19. Procédé selon l'une quelconque des revendications précédentes 14 à 18, dans lequel chaque aiguille a une longueur d'au moins environ 10 µm

20. Procédé selon l'une quelconque des revendications précédentes 14 à 19, dans lequel ladite sonde comprend trois dites électrodes équipées aiguilles, les aiguilles d'une première électrode et d'une deuxième électrode étant espacées latéralement d'une première distance les unes des autres et les aiguilles des première et troisième électrodes étant espacées latéralement d'une deuxième distance les unes des autres, l'étape consistant à faire passer un courant électrique comprenant les étapes consistant à faire passer séparément un courant électrique entre les première et deuxième électrodes et les première et troisième électrodes pour obtenir des première et deuxième valeurs d'impédance de la peau ou un mélange de valeurs d'impédance de la peau entre les valeurs obtenues en utilisant les première et deuxième électrodes et les valeurs obtenues en utilisant les première et troisième électrodes.

21. Procédé selon la revendication 20, dans lequel ladite première distance et ladite deuxième distance sont différentes l'une de l'autre.

22. Procédé selon la revendication 20 ou 21, dans lequel ladite première distance est comprise entre environ 0,1 mm et environ 40 mm.

23. Procédé selon la revendication 22, dans lequel ladite deuxième distance est comprise entre environ 1 mm et 50 mm.

24. Procédé selon l'une quelconque des revendications précédentes 14 à 23, dans lequel dans lequel chacune desdites aiguilles a une longueur allant jusqu'à environ 250 µm.

25. Procédé selon l'une quelconque des revendications précédentes 14 à 24, dans lequel un diamètre extérieur de chacune desdites aiguilles est compris entre environ 20 µm et environ 50 µm.

26. Procédé selon l'une quelconque des revendications précédentes 14 à 25, dans lequel ledit passage comprend le fait de faire passer ledit courant électrique dans les électrodes à une pluralité de fréquences réparties de manière logarithmique dans une plage de 1 kHz à 1 MHz.
